Europäisches Patentamt

European Patent Office

Office européen des brevets

(19)

(11) Veröffentlichungsnummer : **0 010 697**
**B1**

(12)

# EUROPÄISCHE PATENTSCHRIFT

(45) Veröffentlichungstag der Patentschrift :
**11.05.83**

(51) Int. Cl.³ : **C 07 D263/34**

(21) Anmeldenummer : **79104030.6**

(22) Anmeldetag : **18.10.79**

(54) **Verfahren zur Herstellung eines Oxazols.**

(30) Priorität : **02.11.78 DE 2847625**
**09.08.79 CH 7309/79**

(43) Veröffentlichungstag der Anmeldung :
**14.05.80 Patentblatt 80/10**

(45) Bekanntmachung des Hinweises auf die Patenterteilung : **11.05.83 Patentblatt 83/19**

(84) Benannte Vertragsstaaten :
**AT BE CH DE FR GB IT NL SE**

(56) Entgegenhaltungen :
**DE A 2 535 310**
**GB A 532 938**
**GB A 1 081 838**

**Die Akte enthält technische Angaben, die nach dem
Eingang der Anmeldung eingereicht wurden und die
nicht in dieser Patentschrift enthalten sind.**

(73) Patentinhaber : **F. HOFFMANN-LA ROCHE & CO.
Aktiengesellschaft**

**CH-4002 Basel (CH)**

(72) Erfinder : **Hoffmann-Paquotte, Hans Dr.
Erstelweg 14
D-7851 Inzlingen (DE)**

(74) Vertreter : **Lederer, Franz, Dr. et al
Patentanwälte Dr. Franz Lederer Reiner F. Meyer
Lucile-Grahn-Strasse 22
D-8000 München 80 (DE)**

EP 0 010 697 B1

# 0 010 697

## Verfahren zur Herstellung eines Oxazols

Die vorliegende Erfindung betrifft ein neues Verfahren zur Herstellung des 5-Cyan-4-methyl-oxazols(1,3), welches ein wichtiges Zwischenprodukt in der Herstellung von Vitamin $B_6$ darstellt.

Aus der DE-A 25 35 310 ist ein Verfahren zur Herstellung des 5-Cyan-4-methyl-oxazols(1,3) bekannt, wobei man das 5-Carbamoyl-4-methyl-oxazol(1,3) mit einem Niederalkancarbonsäureanhydrid umsetzt und das Reaktionsgemisch oder das aus letzterem isolierte 5-(N-Niederalkanoyl-carbamoyl)-4-methyl-oxazol(1,3) einer Pyrolyse unterwirft. Man arbeitet hierbei ohne Katalysator.

Aus der GB-A 532 938 ist es bekannt, ein aliphatisches Nitril, nämlich Adipinsäurenitril, durch Umsetzung von Essigsäureanhydrid mit Adipinsäureamid in Gegenwart von Antimon, Zink, Zinn, Molybdän, Kobalt, Kupfer, Quecksilber, Nickel oder einem Derivat dieser Metalle als Katalysator herzustellen. Der Katalysator wird dabei in einer Menge von etwa 1 Mol.-%, bezogen auf das Amid, eingesetzt.

Nach dem in der GB-A 1 081 838 offenbarten Verfahren zur Herstellung aromatischer Nitrile durch Umsetzung eines aromatischen Carboxamids mit einem aliphatischen Carbonsäureanhydrid in Anwesenheit von Kupfer-, Zink-, Mangan-, Eisen-, Kobalt-, Nickel- oder Cadmiumsalzen einer schwachen Säure als Katalysator soll der Katalysator in einer Menge von 2-15 Mol.-%, insbesondere 6-12 Mol.-%, eingesetzt werden. Weiterhin soll dem Reaktionsgemisch eine Carbonsäure, vorzugsweise die dem verwendeten Anhydrid entsprechende Carbonsäure, zugesetzt werden, um besonders gute Resultate zu erzielen.

Bisher wurde 5-Cyan-4-methyl-oxazol(1,3) durch Reaktion von auf Schmelztemperatur erhitztem Phosphorpentoxyd mit 5-Carbamoyl-4-methyl-oxazol(1,3) erhalten. Diesem bekannten Verfahren haften jedoch verschiedene Nachteile an. Insbesondere ist die Ausbeute bei diesem bekannten Verfahren verhältnismässig gering, da auf Grund der schlechten Wärmeübertragung sehr leicht Verkohlung eintritt.

Eine Verbesserung dieses bekannten Verfahrens besteht darin, dass die Reaktion des Phosphorpentoxyds mit 5-Carbamoyl-4-methyl-oxazol(1,3) unter Zusatz eines Lösungsmittels, nämlich Chinolin, durchgeführt wird. Dieses bekannte Verfahren hat jedoch wiederum den Nachteil, dass Chinolin unter den Reaktionsbedingungen zu wenig beständig, von unangenehmen Geruch und gesundheitsschädlich ist. Ferner sind die Verfahren zur Regenerierung des Chinolins und zur Aufarbeitung der Folgeprodukte des Phosphorpentoxyds zu einem umweltfreundlichen Produkt aufwendig und mit einer Reihe von technologischen Problemen belastet. Ausserdem sind sowohl Chinolin als auch Phosphorpentoxyd teure und auf dem Markt knappe Rohstoffe.

Eine weitere Verbesserung in der Herstellung des 5-Cyan-4-methyl-oxazols(1,3) besteht darin, dass man das 5-Carbamoyl-4-methyl-oxazol(1,3) mit einem Niederalkancarbonsäureanhydrid umsetzt und das Reaktionsgemisch oder das aus letzterem isolierte 5-(N-Niederalkanoyl-carbamoyl)-4-methyl-oxazol(1,3) einer Pyrolyse unterwirft. Auch dieses pyrolytische Verfahren hat jedoch noch gewisse Nachteile wie insbesondere das Arbeiten bei hohen Temperaturen, grosse Werkstoffprobleme beim Reaktor, Bildung von schwer rezyklisierbaren Nebenprodukten und dergleichen.

Die vorliegende Erfindung zielt daher auf die Schaffung eines Verfahrens ab, welches es ermöglicht, 5-Cyan-4-methyl-oxazol(1,3) in einfacher und billiger Weise, ausgehend von billigen und leicht zugänglichen Rohprodukten, herzustellen, wobei die weiter oben aufgezählten Nachteile der bekannten Verfahren nicht auftreten und wobei das gewünschte 5-Cyan-4-methyl-oxazol(1,3) in hoher Ausbeute und guter Qualität anfällt.

Das obige Ziel wird gemäss der vorliegenden Erfindung dadurch erreicht, dass man das 5-Carbamoyl-4-methyl-oxazol(1,3) mit einem Niederalkancarbonsäureanhydrid in Gegenwart eines Nickel- oder Kupferkatalysators bei Siedetemperatur des Reaktionsgemisches umsetzt und die dabei gebildete Alkancarbonsäure kontinuierlich aus dem Reaktionsgemisch entfernt.

Als Niederalkancarbonsäureanhydride kommen die symmetrischen oder die gemischten Anhydride der geradkettigen oder verzweigten Alkancarbonsäuren mit bis zu 7 Kohlenstoffatomen in Frage. Beispiele solcher Anhydride sind Acetanhydrid, Propionsäureanhydrid, Isopropionsäureanhydrid, Buttersäureanhydrid, n-Valeriansäureanhydrid, das gemischte Anhydrid von Ameisensäure und Essigsäure und dergleichen. Vorzugsweise verwendet man symmetrische Niederalkancarbonsäureanhydride, insbesondere die symmetrischen Anhydride der Alkancarbonsäuren mit bis zu 5 Kohlenstoffatomen. Besonders bevorzugt ist Acetanhydrid.

Als Nickel- bzw. Kupferkatalysator können sowohl metallisches Nickel oder Kupfer sowie auch Nickel- oder Kupferverbindungen, insbesondere Ni(II)- oder Cu(II)-Verbindungen wie beispielsweise die Carbonate, insbesondere basische Carbonate, Oxyde, Hydroxyde, Halogenide insbesondere die Chloride, Formiate, Acetate usw. verwendet werden. Bevorzugt sind im Reaktionsmedium gut lösliche Katalysatoren ; besonders bevorzugt sind jedoch Nickel- und Kupferacetat.

Die Umsetzung des 5-Carbamoyl-4-methyl-oxazols(1,3) mit einem Niederalkancarbonsäureanhydrid erfolgt zweckmässig mit einem etwa 3- bis 10-molaren Ueberschuss an Anhydrid. Vorzugsweise erfolgt die Umsetzung mit einem etwa 3- bis 7-molaren Ueberschuss und besonders bevorzugt mit einem 4- oder 5-molaren Ueberschuss an Anhydrid.

Die Umsetzung erfolgt bei der Siedetemperatur des Reaktionsgemisches. Um eine Rückreaktion der bei der Umsetzung entstehenden Alkancarbonsäure und gebildetem Endprodukt zu verhindern, wird die

2

**0 010 697**

gebildete Alkancarbonsäure kontinuierlich aus dem sich bei siedetemperatur befindenden Reaktionsgemisch entfernt. Dies kann am besten dadurch erfolgen, dass man die gebildete Alkancarbonsäure zusammen mit überschüssigem Anhydrid und gebildetem 5-Cyan-4-methyl-oxazol(1,3) abdestilliert und das Destillat sofort abkühlt. Das Endprodukt 5-Cyan-4-methyl-oxazol(1,3) kann nach beendeter Umsetzung durch fraktionierte Destillation aus dem Destillat isoliert werden. Weitere Mengen Endprodukt können aus dem Rückstand der Umsetzung isoliert werden. Dies erfolgt zweckmässig indem man das als Nebenprodukt entstandene 5-(N-Alkanoyl-carbamoyl)-4-methyl-oxazol(1,3) mit Methanol spaltet und das erhaltene 5-Carbamoyl-4-methyl-oxazol(1,3) wieder wie vorhergehend beschrieben umsetzt.

Das erfindungsgemässe Verfahren kann kontinuierlich oder diskontinuierlich, vorzugsweise kontinuierlich, durchgeführt werden.

Gemäss einer bevorzugten Ausführungsform des erfindungsgemässen Verfahrens wird 5-Carbamoyl-4-methyl-oxazol(1,3) mit einem 5-molaren Ueberschuss an Acetanhydrid unter Zusatz von 0,01 Mol. Nickelacetat als Katalysator bei Siedetemperatur des Gemisches umgesetzt. Nach raschem Abdestillieren wird das Destillat abgekühlt.

In den nachfolgenden Beispielen sind alle %-Angaben Gew.%.

## Beispiel 1

In einem 1 Liter-Dreihalskolben mit Rührer, Thermometer und Destillationsbrücke wurden 128 g 98,5 %iges (1 Mol.) 5-Carbamoyl-4-methyl-oxazol(1,3), 510,5 g (5 Mol.) Acetanhydrid und 1,8 g (0,01 Mol.) Nickelacetat gegeben. Der Ansatz wurde mit einem Heizpilz (450 Watt) so schnell wie möglich aufgeheizt (ca. 12 Minuten). Bei 120-125 °C in der Lösung entstand eine klare Lösung, bei 136-137 °C Sumpftemperatur und 131-132 °C Kopftemperatur begann die Lösung zu sieden. Nun wurde innerhalb von 20 Minuten vom Inhalt des Reaktionskolbens abdestilliert, bis eine Sumpftemperatur von 160 °C erreicht war. Dann wurde die Heizung weggenommen und der Rest des Destillats durch langsames Evakuieren der Apparatur bis zum vollen Wasserstrahlvakuum abdestilliert. Am Ende der Destillation wurde nochmal mit einem Oelbad von 100 °C bis auf eine Sumpftemperatur von 100 °C aufgeheizt. Man erhielt 626,4 g eines farblosen Destillats und 14,2 g eines hellbraunen Rückstandes. Der Gehalt des Destillats an 5-Cyan-4-methyl-oxazol(1,3) betrug 15,8 %, entsprechend 99,0 g. Dies entspricht einer chemischen Ausbeute im Destillat von 91,5 %, bezogen auf eingesetztes 5-Carbamoyl-4-methyl-oxazol (1,3).

## Beispiel 2

In zu Beispiel 1 analoger Weise wurden weitere 13 Ansätze durchgeführt, deren Ergebnisse in der folgenden Tabelle aufgeführt sind. (CMO = 5-Cyan-4-methyl-oxazol(1,3)).

(Siehe die Tabelle, Seite 4)

3

Tabelle

| Versuch Nr. | 1 | 2 | 3 | 4 | 5 | 6 | 7 | 8 | 9 | 10 | 11 | 12 | 13 | 1-13 |
|---|---|---|---|---|---|---|---|---|---|---|---|---|---|---|
| Destillat (g) | 625,7 | 626,0 | 627,1 | 626,0 | 626,8 | 626,8 | 626,5 | 626,0 | 625,9 | 626,2 | 626,3 | 626,5 | 626,8 | 8.142,6 |
| CMO-Gehalt (%) | 15,7 | 15,55 | 15,6 | 15,7 | 15,95 | 16,05 | 15,85 | 15,85 | 16,2 | 16,25 | 15,75 | 15,8 | 15,9 | 15,8 +) |
| CMO-Ausbeute (g) | 98,2 | 97,3 | 97,8 | 98,3 | 100,0 | 100,6 | 99,3 | 99,2 | 101,4 | 101,8 | 98,6 | 99,0 | 99,7 | 1.286,5 |
| Chemische Ausbeute (%) | 90,8 | 90,0 | 90,5 | 90,9 | 92,5 | 93,0 | 91,9 | 91,8 | 93,8 | 94,2 | 91,2 | 91,6 | 92,2 | 91,5 |
| Rückstände (g) | 14,7 | 14,8 | 13,5 | 14,8 | 14,1 | 13,8 | 14,2 | 14,9 | 14,0 | 13,8 | 13,8 | 14,1 | 14,3 | 184,8 |

+) Durchschnitt aus 3 Analysen (15,9 ; 15,8 ; 15,7)

**0 010 697**

Die chemische Ausbeute betrug also nach der Analyse der vereinigten Destillate aus 13 gleichartigen Ansätzen 91,5 % d. Th. [bezogen auf eingesetztes 5-Carbamoyl-4-methyloxazol (1,3)].

### Aufarbeitung der Rückstände

Der Rückstand jedes einzelnen Ansatzes wurde mit jeweils 170 ml Methanol versetzt und 10 Minuten unter Rückfluss gekocht. Dabei zersetzte sich das im Rückstand vorhandene 5-(N-Acetyl-carbamoyl)-4-methyl-oxazol(1,3) quantitativ unter Rückbildung von 5-Carbamoyl-4-methyl-oxazol(1,3) und Bildung von Essigsäuremethylester.

Die gesammelten, mit Methanol behandelten und gelösten Rückstände wurden, zuerst bei Normaldruck und dann im Vakuum der Wasserstrahlpumpe bis zur Trockene eingeengt.

Ausbeute : 145,5 g = 78,7 % der eingesetzten Menge.

Die 145,5 g wurden mit 510,5 g Acetanhydrid und 1,8 g Nickelacetat in zu Beispiel 1 analoger Weise umgesetzt. Man erhielt 603,2 g Destillat, mit einem Gehalt an 5-Cyan-4-methyl-oxazol(1,3) von 12,8 % entsprechend 77,2 g. Die Gesamtausbeute an Oxazolnitril im Destillat der 14 Ansätze betrug also 1 363,7 g. Dies entspricht einer chemischen Ausbeute von 97,0 % d. Th.

### Beispiel 3

In einem 1 Liter-Dreihalskolben, versehen mit Rührer, Thermometer und Destillationsbrücke wurden 128 g (1 Mol.) 98,5 %iges 5-Carbamoyl-4-methyl-oxazol(1,3), 510,5 g Essigsäureanhydrid (5 Mol.) und 2 g (0,01 Mol.) Kupferacetat $\cdot$ $H_2O$ mit einem 450-Watt-Heizpilz so schnell wie möglich aufgeheizt (ca. 12 Minuten). Bei 120-125 °C in der Lösung entstand eine klare grasgrüne Lösung. Bei 138 °C Sumpftemperatur und 132 °C Kopftemperatur begann die Lösung zu sieden. Nun wurde innerhalb von 20 Minuten vom Inhalt des Reaktionskolbens abdestilliert bis eine Sumpftemperatur von 160 °C erreicht war. Dann wurde die Heizung weggenommen und der Rest des Destillats durch langsames Evakuieren der Apparatur bis zum vollen Wasserstrahlvakuum abdestilliert. Am Ende der Destillation wurde nochmal mit einem Oelbad von 100 °C bis auf eine Sumpftemperatur von 100 °C aufgeheizt. Man erhielt 614,3 g eines farblosen Destillates und 25,4 g eines braunschwarzen Rückstandes. Der Gehalt des Destillates an 5-Cyan-4-methyl-oxazol(1,3) betrug 14,4 %, entsprechend 88,5 g. Dies entspricht einer chemischen Ausbeute von 81,9 %, bezogen auf eingesetztes 5-Carbamoyl-4-methyl-oxazol(1,3).

### Beispiel 4

In zu Beispiel 1 analoger Weise wurden die folgenden Versuche mit verschiedenen Katalysatoren durchgeführt, deren Ergebnisse in der nachfolgenden Tabelle aufgeführt sind. [CMO = 5-Cyan-4-methyl-oxazol(1,3)].

Ansatz :

126,1 g (1 Mol) 5-Carbamoyl-4-methyl-oxazol(1,3)

510,5 g (5 Mol) Acetanhydrid

+ 0,01 Mol Katalysator

(Siehe die Tabelle, Seite 6)

5

Tabelle

| Versuch Nr. | Katalysator | g | Destillations-rückstand (g) | Destillat | | | Ausbeute CMO % |
|---|---|---|---|---|---|---|---|
| | | | | g | %CMO | gCMO | |
| 1 | $Ni(OAc)_2 \cdot 2H_2O$ | 2,5 | 13,4 | 625,3 | 15,85 | 99,1 | 99,7 |
| 2 | $NiCO_3 \cdot 2NiOH + 4H_2O$ | 1,3* | 16,4 | 620,7 | 15,30 | 95,0 | 87,9 |
| 3 | $NiO$ | 0,7 | 18,6 | 619,4 | 15,25 | 94,5 | 87,4 |
| 4 | $Ni(COOH)_2 \cdot 2H_2O$ | 1,9 | 21,0 | 616,1 | 15,0 | 92,4 | 85,5 |

* Hier 1/300 Mol eingesetzt, da 3 $Ni^{++}$/Mol Katalysator

# 0 010 697

## Ansprüche

1. Verfahren zur Herstellung des 5-Cyan-4-methyloxazols(1,3), dadurch gekennzeichnet, dass man das 5-Carbamoyl-4-methyl-oxazol(1,3) mit einem Niederalkancarbonsäureanhydrid in Gegenwart eines Nickel- oder Kupferkatalysators bei Siedetemperatur des Reaktionsgemisches umsetzt und die dabei gebildete Alkancarbonsäure kontinuierlich aus dem Reaktionsgemisch entfernt.

2. Verfahren nach Anspruch 1, dadurch gekennzeichnet, dass man die Umsetzung mit einem etwa 3- bis 10-molaren Ueberschuss, vorzugsweise mit einem etwa 3- bis 7-molaren Ueberschuss und insbesondere mit einem 4- oder 5-molaren Ueberschuss an Niederalkancarbonsäureanhydrid durchführt.

3. Verfahren nach Anspruch 1 oder 2, dadurch gekennzeichnet, dass man als Niederalkancarbonsäureanhydrid Acetanhydrid verwendet.

4. Verfahren nach Anspruch 1, 2 oder 3, dadurch gekennzeichnet, dass man als Katalysator einen im Reaktionsmedium gut löslichen Katalysator verwendet.

5. Verfahren nach einem der Ansprüche 1-4, dadurch gekennzeichnet, dass man als Katalysator eine Ni(II)- bzw. Cu(II)-Verbindung verwendet.

6. Verfahren nach Anspruch 4 oder 5, dadurch gekennzeichnet, dass man als Katalysator Nickelacetat oder Kupferacetat verwendet.

7. Verfahren nach einem der Ansprüche 1-6, dadurch gekennzeichnet, dass man die Umsetzung bei der Siedetemperatur des Reaktionsgemisches durchführt, rasch abdestilliert und das Destillat abkühlt.

8. Verfahren nach einem der Ansprüche 1-7, dadurch gekennzeichnet, dass man dieses Verfahren in kontinuierlicher Weise durchführt.

## Claims

1. Process for the manufacture of 5-cyano-4-methyl-oxazole(1,3), characterized by reacting 5-carbamoyl-4-methyl-oxazole(1,3) with a lower alkanecarboxylic acid anhydride in the presence of a nickel or copper catalyst at the boiling temperature of the reaction mixture and continuously removing the thereby formed alkanecarboxylic acid from the reaction mixture.

2. Process according to claim 1, characterized in that the reaction is carried out with an about 3- to 10-molar excess, preferably with an about 3- to 7-molar excess and especially with a 4- or 5-molar excess, of lower alkanecarboxylic acid anhydride.

3. Process according to claim 1 or 2, characterized in that acetic anhydride is used as the lower alkanecarboxylic acid anhydride.

4. Process according to claim 1, 2 or 3, characterized in that a catalyst which has a good solubility in the reaction medium is used as the catalyst.

5. Process according to any one of claims 1-4, characterized in that a Ni (II) or Cu (II) compound is used as the catalyst.

6. Process according to claim 4 or 5, characterized in that nickel acetate or copper acetate is used as the catalyst.

7. Process according to any one of claims 1-6, characterized in that the reaction is carried out at the boiling temperature of the reaction mixture, the mixture is rapidly distilled and the distillate is cooled.

8. Process according to any one of claims 1-7, characterized in that this process is carried out in a continuous manner.

## Revendications

1. Procédé pour la préparation du 5-cyano-4-méthyl-oxazole-(1,3), caractérisé en ce que l'on fait réagir le 5-carbamoyl-4-méthyl-oxazole-(1,3) avec une anhydride d'acide alcanoïque inférieur en présence d'un catalyseur au nickel ou au cuivre à la température d'ébullition du mélange de réaction en éliminant en continu l'acide alcanoïque formé du mélange de réaction.

2. Procédé selon la revendication 1, caractérisé en ce que l'on effectue la réaction avec un excès d'environ 3 à 10 moles par mole, de préférence d'environ 3 à 7 moles par mole et plus spécialement de 4 ou 5 moles par mole en anhydride d'acide alcanoïque inférieur.

3. Procédé selon la revendication 1 ou 2, caractérisé en ce que l'on utilise en tant qu'anhydride d'acide alcanoïque inférieur l'anhydride acétique.

4. Procédé selon la revendication 1, 2 ou 3, caractérisé en ce que l'on utilise en tant que catalyseur un catalyseur bien soluble dans le milieu de réaction.

5. Procédé selon l'une des revendications 1 à 4, caractérisé en ce que l'on utilise en tant que catalyseur un composé du Ni-(II) ou du Cu-(II).

6. Procédé selon la revendication 4 ou 5, caractérisé en ce que l'on utilise en tant que catalyseur l'acétate de nickel ou l'acétate de cuivre.

7. Procédé selon l'une des revendications 1 à 6, caractérisé en ce que l'on effectue la réaction à la température d'ébullition du mélange de réaction, on distille rapidement et on refroidit le distillat.

8. Procédé selon l'une des revendications 1 à 7, caractérisé en ce que ce procédé est mis en œuvre en continu.

7